# EUROPÄISCHE PATENTSCHRIFT

(11) **EP 0 918 464 B1**
(45) Veröffentlichungstag und Bekanntmachung des Hinweises auf die Patenterteilung: **04.10.2001**
(21) Anmeldenummer: 97924895.2
(22) Anmeldetag: 16.05.1997
(51) Int. Cl.: A23C 9/20, A23C 9/142, A61K 35/20

(54) **VERFAHREN ZUR HERSTELLUNG VON KOLOSTRALMILCHPRODUKTEN SOWIE DEREN VERWENDUNG**
PROCESS FOR PRODUCTION OF COLOSTRAL MILK PRODUCTS AND USE THEREOF
PROCEDE DE PRODUCTION DE PRODUITS TIRES DU LAIT COLOSTRAL ET UTILISATION DE CES PRODUITS

(30) Priorität: 17.05.1996 DE 19619990
(43) Veröffentlichungstag der Anmeldung: 02.06.1999
(73) Patentinhaber: Adler, Charlotte, 86161 Augsburg (DE); Achenbach, Michael, 94501 Aidenbach (DE)
(72) Erfinder: Adler, Charlotte, 86161 Augsburg (DE); Achenbach, Michael, 94501 Aidenbach (DE)
(74) Vertreter: Winter, Brandl & Partner
(86) Internationale Anmeldenummer: DE9701003
(87) Internationale Veröffentlichungsnummer: WO9743905

(56) Entgegenhaltungen:
- EP-A- 0 173 999
- EP-A- 0 334 776
- EP-A- 0 471 890
- EP-A- 0 652 013
- WO-A-93/08264
- WO-A-95/00155
- WO-A-95/10192
- GB-A- 2 289 278

## Beschreibung

Die vorliegende Erfindung betrifft ein Verfahren zur Herstellung entkaseinierter Kolostralmilchprodukte gemäß Anspruch 1, ein Kolostralmilchprodukt gemäß Anspruch 10, ein Getränk, welches Kolostralmilchprodukte enthält gemäß Anspruch 15, sowie die Verwendung von Kolostralmilchprodukten gemäß den Ansprüchen 16 und 17.

Es ist seit längerem bekannt, daß das Kolostrum - also die Vormilch - aller Säugetiere, einschließlich des Menschen, wertvolle Bestandteile enthält, die die Neugeborenen vor Infektionen schützen und weitere günstige Wirkungen aufweisen.

So beschreibt beispielsweise die EP-A-0 173 999 ein Verfahren zur Herstellung von Immunglobulinen aus Rinder- oder Humankolostralmilch, wobei eine Immungfobulinfraktion aus Rinder- oder Humankolostralmilch gewonnen wird, die sowohl in der Humanmedizin als auch in der Veterinärmedizin zur Behandlung oder Prophylaxe von bakteriellen und viralen Darminfektionen geeignet ist.

So hat sich beispielsweise herausgestellt, daß die Kolostralimmunglobuline gegen die Bakterien: Escherichia coli, Pseudomonas aeruginosa, Klebsiella, Staphylokokken, Enterokokken und Streptokokken sowie gegen die Viren: Varicellavirus, Cytomegalievirus, Rubellavirus, Herpesvirus und Rotavirus wirksam waren.

Hergestellt wurde eine derartige Kolostralglobulinfraktion durch Aufarbeiten von Kolostralmilch unter Ausfällen der Kaseine bei einem pH-Wert von 4,0 bis 5,5 und anschließendem Abfiltrieren des Kaseinkuchens durch Tangentialfiltration und nachfolgender Ultrafiltration mit einer Trenngrenze von 5 000 bis 80 000 Dalton.

Da der Keimgehalt in der rohen Kolostralmilch bei 10⁶ bis 10⁸ Keimen pro Milliliter liegen kann, beschäftigt sich die EP-A-0 471 890 mit einem Verfahren zur Gewinnung einer sterilfiltrierten, kaseinhaltigen Kolostralmilch, bei welchem das Rohkolostrum soweit angesäuert wird, daß das zunächst ausfallende Kasein wieder in Lösung geht und die erhaltene Lösung anschließend sterilfiltriert wird.

Dabei geht die Lehre der EP-A-0 471 890 davon aus, daß das Kasein selbst günstige therapeutisch anwendbare Eigenschaften hat, die besonders die Wirkung der Immunglobuline bei gastrointestinalen Störungen unterstützen. Gemäß diesem Stand der Technik werden beispielsweise aus dem Kasein opiatartige Wirkstoffe freigesetzt, die zur Hemmung der Darmbewegung und Förderung der Elektrolyt- und Wasserresorption führen. Daher ist es nach der Lehre der EP-A-0 471 890 wünschenswert, die Kolostralmilch möglichst wenig in ihrer Proteinzusammensetzung zu verändern, da sie geradezu optimal für die Prophylaxe und Therapie von gastrointestinalen Infektionen und Störungen zusammengesetzt ist.

Darüber hinaus beschreibt die WO 95/10192 ein Nährgetränk, auf Kolostrumbasis, welches die körperliche Leistungsfähigkeit und Erholung verbessert.

Ein derartiges Getränk wird hergestellt durch Entfetten des Kolostrums und anschließender Fällung und Abfiltrieren des Kaseins und Sterilfiltrieren der erhaltenen Kolostrummolke.

Ein derartiges Kolostralmilchprodukt hat jedoch den Nachteil, daß aufgrund der Kaseinpräzipitation viele günstige Bestandteile des Kolostrums wie etwa der Gehalt an Wachstumsfaktoren, beispielsweise IGF-1, IGF-2 oder TGF-β vermindert ist und daher die Ausbeute eines derartigen Kolostralmilchproduktes bezogen auf das Rohkolostrum nur dürftig ausfällt, ja bisweilen bestimmte Wachstumsfaktoren wie IGF-2 und TGF-β überhaupt nicht mehr nachgewiesen werden können.

Ausgehend von diesem Stand der Technik war es daher Aufgabe der vorliegenden Erfindung, ein Verfahren zur Verfügung zu stellen, welches die Herstellung eines weitgehend entkaseinierten Kolostralmilchproduktes erlaubt, welches insbesondere die im Kolostrum enthaltenen Wachstumsfaktoren möglichst quantitativ enthalten.

Die Lösung dieser Aufgabe erfolgt in verfahrenstechnischer Hinsicht durch die Merkmale des Anspruchs 1, bezüglich eines Produktes durch die Merkmale der Ansprüche 10 und 15, und verwendungstechnisch durch die Merkmale der Ansprüche 16 und 17.

Dadurch, daß das Kasein derart angesäuert wird, daß es in Lösung bleibt und anschließend eine Ultrafiltration mit einer Ausschlußmolekularmasse von ca. 10⁶ Dalton durchgeführt wird, wird einerseits das Rohkolostrum von Kasein befreit. Dies ist insbesondere für Allergiker und für Neurodermitiker erwünscht, da Kasein für diesen Personenkreis unerwünschte Nebenwirkungen haben kann. Andererseits erreicht man überraschenderweise durch die Ultrafiltration einen Gehalt von bis zu ca. 95% bezogen auf den Gehalt des Rohkolostrums an Wachstumsfaktoren, insbesondere an IGF-1.

Es ist bei dem erfindungsgemäßen Verfahren insbesondere überraschend, daß Kasein bei einer Molekularmasse von ca. 150·10⁶ Dalton und mit seiner riesigen Oberfläche im Wege einer Ultafiltration abgetrennt werden kann, wobei die erwünschten niedermolekularen Bestandteile des Kolostrums, wie z. B. Wachstumsfaktoren und Peptide, nahezu quantitativ in das Filtrat übertreten - also ohne an die riesige Proteinoberfläche des Kaseins gebunden zu werden.

Ein mittels des erfindungsgemäßen Verfahren hergestelltes Kolostralmilchprodukt ist für viele Anwendungen im therapeutischen und Lebensmittelergänzungsbereich nützlich. So hat sich beispielsweise bei der Untersuchung von Hochleistungssportlern ergeben, daß die Kreatinkinase im Serum, ein Indikator für die körperliche Belastung, signifikant niedriger ist, wenn die Probanden das erfindungsgemäße Kolostralmilchprodukt in fester oder flüssiger Form eingenommen hatten, sowohl regelmäßig über einen längeren Zeitraum oder unmittelbar vor starker körperlicher Beanspruchung.

Zu diesem Mechanismus ist zu sagen - ohne hierauf beschränkt zu sein, - daß die Kreatinkinase immer dann von Muskelzellen freigesetzt wird, wenn Muskelzellen in irgendeiner Form geschädigt sind. Eine derartige Schädigung tritt beispielsweise nach extremen körperlichen Belastungen auf, oder auch bei pathologischen Erscheinungsbildern wie beispielsweise Herzinfarkt oder Muskelerkrankungen. Mittlerweile ist in mehreren Studien gezeigt worden, darunter eine Doppelblindstudie während der Olympischen Winterspiele in Lillehammer mit dem finnischen Skiteam, daß Kolostrummilchprodukte die Konzentration der freien Kreatinkinase im Serum um 30 bis 40 Prozent erniedrigen, wodurch die Leistungsfähigkeit signifikant länger erhalten bleibt und sich die Regenerationszeit, also das Refraktärstadium der Muskulatur, merklich verkürzt.

Desweiteren ist aus in vitro-Studien bekannt, daß das Kolostrum die Lebensfähigkeiten und das Wachstum verschiedener Zelltypen in der Zellkultur unterstützt, da es im wesentlichen vier Hauptfraktionen aufweist, nämlich:
eine Wachstumsfaktorfraktion, eine Immunglobolinfraktion, eine Enzymproteinfraktion sowie eine Vitamin- und Peptidfraktion.

Es ist ferner aus Untersuchungen des Kolostrums bekannt, daß diese wesentlichen Bestandteile des Kolostrums im wesentlichen bei der Geburt des Kalbes ihren Höhepunkt erreichen und bereits 6-12 Stunden nach der Geburt bereits schon über die Hälfte ihres Gehaltes an wesentlichen Bestandteilen verloren hat und danach über die nächsten Tage stetig abfällt, wobei nach 3-4 Tagen nur noch Spuren vorhanden sind.

Es hat sich ferner herausgestellt, daß Kolostrumprodukte ohne weiteres bei sportlichen Veranstaltungen den Athleten gegeben werden können, da sie keinerlei Dopingsubstanzen enthalten.

Darüber hinaus hat sich herausgestellt, daß das erfindungsgemäße Kolostralmilchprodukt als Arzneimittel, Nahrungsergänzungsmittel oder Kosmetikum sowie als Zusatz für Arzneimittel, Nahrungsergänzungsmittel, Getränke oder Kosmetika eingesetzt werden kann.

Neben der Anwendung im Sport- und Extremsportbereich haben sich überragende therapeutische Erfolge bei der Behandlung der Neurodermitis ergeben. Hierzu wurde das erfindungsgemäße Kolostralmilchprodukt bevorzugt in Liposomen eingeschlossen und zu einer Cremegrundlage gemischt und auf die befallenen Hautpartien der an Neurodermitis leidenden Patienten aufgebracht.

Zusätzlich wurde das erfindungsgemäße Kolostralmilchprodukt in Form eines Getränkes verabreicht.

Das Patientenkollektiv umfaßte 20 an Neurodermitis leidende Personen im Erwachsenenalter, die an neurodermitischen Erscheinungen, insbesondere auch in den Ellenbogen, litten. Die Patienten nahmen keinerlei Medikamente ein, sondern behandelten die befallenen Hautareale mit einer Creme, welche das erfindungsgemäße Kolostralmilchprodukt enthielt. Die Konzentration des Kolostralmilchproduktes in der Creme betrug ca. 10 Gew.-%. Als Cremegrundlage diente z.B. Soja-Öl, Tegomuls, Vitamin E + A, Cholesterol, HSA, Contrex, Carotin DF + HF, Orangenblüten-Öl, Melissen-Öl, Mandarinen-Öl, Neroli-Öl, Aqua dest.. Alternativ kann die Neurodermitis auch mit einer Liposomencreme behandelt werden, deren Liposomen das erfindungsgemäße Kolostralmilchprodukt enthielten. Die Konzentration der Liposomen in der Cremegrundlage (z.B. Soja-Öl, Tegomuls, Vitamin E + A, Cholesterol, HSA, Contrex, Carotin DF + HF, Orangenblüten-Öl, Melissen-Öl, Mandarinen-Öl, Neroli-Öl, Aqua dest.) betrug ca. 0,1 ml/g oder 10% Liposomen.

Die Behandlung wurde 14 Tage lang durchgeführt und dann vom behandelnden Arzt begutachtet. Hierbei stellte sich heraus, daß 18 von 20 Patienten einen nahezu 90-prozentigen Rückgang der neurodermitischen Hauterscheinungen aufwiesen. Sowohl die Liposomencreme als auch das oral verabreichte Kolostralmilchprodukt-Getränk wurden dann für 14 Tage abgesetzt und die neurodermitischen Exantheme erneut ärztlich begutachtet. Es stellte sich dann heraus, daß sämtliche Patienten mit überwiegendem Zurückgehen der Hautsymptome nach der ersten Kolostralmilchprodukt-Behandlungsperiode jetzt wieder an deutlichem neurodermitischen Ekzem litten.

Hierauf wurde erneut eine Behandlungsperiode von 14 Tagen angesetzt, während dieser die befallenen Hautpartien erneut mit der Creme oder Liposomencreme, die das erfindungsgemäße Kolostralmilchprodukt in Liposomen eingeschlossen enthält, behandelt wurde und unterstützend 10 ml pro Tag eines Kolostralmilchprodukt-Getränkes verabreicht und die Hauterscheinungen erneut ärztlich begutachtet wurden.

Hierbei stellte sich heraus, daß 17 von 20 Probanden, die an der Studie teilnahmen, wieder einen deutlichen Rückgang des neurodermitischen Ekzems aufwiesen.

Aus dieser Cross-over-Studie ist klar erkennbar, daß die Besserung der neurodermitischen Hauterscheinung auf das applizierte, erfindungsgemäße Kolostralmilchprodukt zurückzuführen ist.

Erste Studien über einen längeren Zeitraum von ca. 6 Monaten zeigen auch, daß das erfindungsgemäße Kolostralmilchprodukt, vorzugsweise in Form eines Dermatikums, insbesondere einer Creme, Lotion oder Salbe als Langzeittherapeutikum für die Hauterscheinungen der Neurodermitis geeignet ist.

Es wurde darüber hinaus eine Kontrollstudie mit Placebo-Liposomencreme durchgeführt, wobei die Cremegrundlage dieselbe war wie in der Versuchsgruppe, wobei die Liposomen jedoch lediglich physiologische Kochsalzlösung enthielten. Als Zusatzgetränkgabe wurde entkaseinierte Milch statt der erfindungsgemäßen Kolostralmolke verabreicht.

Innerhalb der Kontrollgruppe von 15 Probanden mit neurodermitischen Hauterscheinungen zeigten lediglich zwei signifikante Besserungen der Hauterscheinungen nach einem 14-tägigen Behandlungsintervall.

Durch dieses placebokontrollierte Experiment ist die Wirksamkeit des erfindungsgemäßen Kolostralmilchproduktes weiter gesichert.

Neben der bereits erwähnten ausdauersteigernden Wirkung und der günstigen Wirkung auf die Erholungsphase der Muskulatur und der oben nachgewiesenen Wirkung bei Neurodermitis, läßt sich das erfindungsgemäße Kolostralmilchprodukt auch noch einsetzen als Bestandteil von Babynahrung, diätetischer Nahrung, klinischer Nahrung, insbesondere Sondennahrung, sowie bei Erkrankungen des rheumatischen Formenkreises, Allergien, insbesondere Heuschnupfen, Pollenallergien, Herzinfarkten und muskulären Erkrankungen während der Rehabilitation. Diese Wirkungen zeigen sich insbesondere an der Senkung der Kreatinkinase, was eine Indikation für eine Erholungsphase der Muskulatur ist. Es hat sich ferner herausgestellt, daß die erfindungsgemäßen Kolostralmilchprodukte sich zur Unterstützung von Leber- und Knochenerkrankungstherapien eignen.

Ferner hat das erfindungsgemäße Kolostralmilchprodukt auch günstige Wirkung zur Prophylaxe und Unterstützung viraler, bakterieller und mykotischer Infektionen sowie generell zur Unterstützung und Stärkung des Immunsystems, was sich durch den erhöhten Gehalt an Immunglobulinen, insbesondere IgG, IgA IgE und IgM erklärt. Weiterhin spielt auch das TGF-β eine nicht unwichtige Rolle im Immunsystem, da TGF-β auf die β-Lymphozyten wirkt und einen Switch von IgE zu IgA bewirkt und eine Wirkung auf die Immunstimulanz hat.

Darüber hinaus hat sich herausgestellt, daß das erfindungsgemäße Kolostralmilchprodukt auch günstige Auswirkungen auf die Wundheilung hat, insbesondere bei postoperativer Nachsorge oder auch kleineren Verletzungen, bei oraler und zusätzlich lokaler topischer Anwendung.

Die Unteransprüche stellen bevorzugte Ausführungsformen der vorliegenden Erfindung dar.

Weitere Vorteile und Merkmale ergeben sich anhand der Beschreibung von Ausführungsbeispielen.

### Beispiel 1

20 Liter entfettete Rinderkolostralmilch, die in den ersten 24 Stunden nach der Geburt des Kalbes gewonnen wurden, wurden bei + 4°C durch Zugabe von 60 mMol EDTA und anschließend von 365 ml HCI auf pH 2,54 gebracht. Diese pH-abgesenkte Rinderkolostralmilch wurde im Anschluß bei einer Ausschlußmolekularmasse von 1 000 000 Da ultrafiltriert, wobei die Temperatur konstant bei + 4°C bis + 8°C gehalten wurde. Es wurde dabei ein Filtermodul der Firma Millipore 1000 KD aus Zelluloseacetat wegen seiner geringen Proteinbindung verwendet. Es wurden dabei 10 L Permeat gewonnen. Die Ausbeute betrug bei dieser Vorgehensweise 50%. Nach der Ultrafiltration wurde das klare Permeat mit 250 ml NaOH auf pH 6,2 angehoben. Die dabei entstandene leichte Trübung wurde durch Zentrifugation beseitigt. Das nun klare Permeat wurde mit 10 L Aqua dest. verdünnt und einer weiteren Ultrafiltration mit einer Ausschlußmolekularmasse von 1000 Da unterzogen, um das bei der pH-Absenkung und anschließenden pH-Anhebung entstandene Salz zu entfernen. Dies hat auch eine gleichzeitige. Entzuckerung zur Folge. Wiederum wurde das Rinderkolostralmilchprodukt konstant bei einer Temperatur von + 4°C bis + 8°C gehalten und solange ultrafiltriert, bis die Ausgangsmenge von 10 Liter Retentat erreicht wurde. Dieses Retentat wurde nach herkömmlicher Methode bei 0,22 µm sterilfiltriert. Es mußten keine Vorfilter verwendet werden. Das Produkt war leicht sterilfiltrierbar. Im Anschluß wurde eine Analyse erstellt.

Das nach dem erfindungsgemäßen Verfahren erhaltenes Kolostralmilchprodukt hatte die folgende analytisch erfaßte Zusammensetzung gemäß Tab. 1.

Besonders auffallend ist der hohe Anteil an Immunglobulinen und Wachstumsfaktoren, insbesondere IGF-1.

Ein derartiges Kolostralmilchprodukt kann direkt als Getränk verwendet werden oder kann zu unterschiedlichen Nahrungsmitteln zugesetzt werden, so beispielsweise auch zu Backwaren, insbesondere zu energiehaltigen Riegeln, bevorzugt zu sogenannten Powerbars. Der Zusatz zu Backwaren oder Riegeln erfolgt bevorzugt nach einer Sprühtrocknung oder einer Gefriertrocknung.

**Tabelle 1**

| | |
|---|---|
| Energiegehalt | 80,0 kJ/100g |
| pH | 6,16 |
| Osmolarität | 264,0 mOsm/kg |
| Gesamtprotein | 0,3 g/l |
| Trockensubstanz | 5,24 g/l |
| Asche | 0,68 mg/100 ml |
| Laktose | 0,2 mg/100 ml |
| Glukose | 10,0 mg/100 ml |
| Gesamtkohlenhydrate | 20,0 g/l |
| Fett | <0,03 mg/100 ml |
| | |

| ***VITAMINE*** | |
|---|---|
| | |
| Vitamin A | <150,0 µg/100 ml |
| Thiamin (Vit. B1) | 63,5 µg/100 ml |
| Riboflavin (Vit. B2) | 620,0 µg/100 ml |
| Pyridoxin (Vit. B6) | 3500,0 µg/100 ml |
| Cobalamin (Vit. B12) | 0,034 µg/100 ml |
| Folsäure | 2,65 µg/100 ml |
| Vitamin C | 270,0 µg/100 ml |
| Cholecalciferol (Vit.D3) | 0,28 µg/100 ml |
| Tocopherol (Vit. E) | 30,0 µg/100 ml |
| Vitamin K | 0,054 µg/100 ml |
| Ubichinon (Vit.Q 10) | 5,4 µg/100 ml |
| | |

| ***IONEN*** | |
|---|---|
| | |
| Natrium (Na) | 943,7 mg/l |
| Kalium (K) | 1883,5 mg/l |
| Kalzium (Ca) | 490,52 mg/l |
| Magnesium (Mg) | 147,98 mg/l |
| Eisen (Fe) | 0,02 mg/l |
| Kupfer (Cu) | 0,004 mg/l |
| Zink (Zn) | 0,30 mg/l |
| Chrom (Cr) | 0,0014 mg/l |
| Phosphor (P) | 498,0 mg/l |
| Selen (Se) | 0,018 mg/l |
| | |

| ***ORGANISCHE MOLEKÜLE*** | |
|---|---|
| | |
| Kreatinin | 17,2 mg/l |
| | |

| ***FREIE AMINOSÄUREN*** | |
|---|---|
| | |
| Alanin | 6,60 mg/l |
| Arginin | 0,0056 mg/l |
| Tryptophan | 2,66 mg/l |
| Cystin | <0,27 mg/l |
| Methionin | 1,03 mg/l |
| Phosphoserin | 1,05 mg/l |
| Taurin | <0,363 mg/l |
| Phosphoethanolamin | 7,31 mg/l |
| Asparaginsäure | 4,85 mg/l |
| Threonin | 0,00014 mg/l |
| Serin | 4,85 mg/l |
| Glutaminsäure | 9,44 mg/l |
| Glutamin | 7,99 mg/l |
| Prolin | 7,37 mg/l |
| Glycin | 4,85 mg/l |
| Alanin | 6,6 mg/l |
| Citrullin | 1,05 mg/l |
| Valin | 11,13 mg/l |
| Isoleucin | 4,85 mg/l |
| Leucin | 9,44 mg/l |
| Tyronsin | 3,26 mg/l |
| Phenylalanin | 4,95 mg/l |
| β-Alanin | <0,27 mg/l |
| β-Aminoisobuttersäure | 1,03 mg/l |
| Ornithin | 0,53 mg/l |
| Lysin | 7,99 mg/l |
| | |

| ***IMMUNGLOBULINE*** | |
|---|---|
| | |
| IgG | 98,0 mg/100 ml |
| IgA | 3,0 mg/100 ml |
| IgM | 2,0 mg/100 ml |
| IgE | 0,026 µg/100 ml |
| | |

| ***NATÜRLICHE WACHSTUMS- FAKTOREN*** | |
|---|---|
| | |
| IGF-1 (Somatomedin C) | 380,0 ng/ml |
| IGF-2 | 242,0 ng/ml |
| TGF-β | 35,0 ng/ml |

### Beispiel 2

Es wurde bei der pH-Absenkung, Ultrafiltration, pH-Anhebung und Zentrifugation wie in Beispiel 1 verfahren. Danach wurden, um die Ausbeute zu vergrößern 10 L Aqua dest. hinzugefügt, die vorher ebenfalls auf pH 2,54 gebracht wurden. Die Ultrafiltration wurde fortgesetzt und es wurden nochmals 10 L Permeat gewonnen. Dieses Permeat wurde zusammen mit dem Permeat der 1. Ultrafiltration, und zusätzlich 10 Litern Aqua dest. mit einer Ausschlußmolekularmasse von 500 Da ultrafiltriert, wobei eine Ultrafiltationsmembran der Firma Amicon verwendet wurde. Auch hier wurde die Temperatur konstant auf + 4°C bis + 8°C gehalten. Es wurde so lange ultrafiltriert, bis 15 L Retentat übrig blieben. Dadurch konnte die Ausbeute auf 75% erhöht werden. Dieses Retentat wurde nach herkömmlicher Methode bei 0,22 µm sterilfiltriert. Es mußten keine Vorfilter verwendet werden. Das Produkt war leicht sterilfiltrierbar. Im Anschluß wurde eine Analyse erstellt. Die Ergebnisse sind in Tabelle 2 gezeigt.

### Vergleichsbeispiel 1

20 L entfettete Kolostralmilch wurden leicht erwärmt (ca. 38°C) und mit 240 ml HCI auf pH 4,5 abgesenkt. Dabei verklumpte das Kasein und war mit einem Sieb und einer anschließenden Zentrifugation leicht zu entfernen. Eine Trübung der Molke war dabei nicht zu verhindern. Anschließend wurde die Molke mit einer Ausschlußmolekularmasse von 1 000 000 Da ultrafiltriert, wobei ein Modul der Firma Millipore verwendet wurde. Es wurden 7 L Permeat gewonnen, die mit 80 ml NaOH auf pH 6,2 gebracht wurden. Es entstand wieder eine leichte Trübung, die mit einer Zentrifugation entfernt werden konnte. Das Permeat wurde nach herkömmlicher Methode bei 0,22 µm sterilfiltriert. Es mußten keine Vorfilter verwendet werden. Das Produkt war leicht sterilfiltrierbar. Im Anschluß wurde eine Analyse erstellt. Die Ergebnisse sind in Tabelle 2 gezeigt.

### Vergleichsbeispiel 2

20 L entfettete Kolostralmilch wurden leicht erwärmt (ca. 38°C) und mit 240 ml HCI auf pH 4,5 abgesenkt. Dabei verklumpte das Kasein und war mit einem Sieb und einer anschließenden Zentrifugation leicht zu entfernen. Anschließend wurde der pH mit 100 ml NaOH wieder auf pH 6,2 gebracht und mit einer Ausschlußmolekularmasse von 1 000 000 Da ultrafiltriert, wobei ein Modul der Firma Millipore verwendet wurde. Es wurde 7 L Permeat gewonnen. Diese wurden im Anschluß nach herkömmlicher Methode bei 0,22 µm sterilfiltriert. Es mußten keine Vorfilter verwendet werden. Das Produkt war leicht sterilfiltrierbar. Im Anschluß wurde eine Analyse erstellt. Die Ergebnisse sind in Tabelle 2 gezeigt.

### Vergleichsbeispiel 3

20 L entfettete Kolostralmilch wurden bei + 4°C bis + 8°C mit 240 ml HCI auf pH 4,5 abgesenkt. Hierbei wurde festgestellt, daß das Kasein nur unzureichend verklumpte. Es konnte keine richtige Molke gewonnen werden. Das geronnene Kasein wurde abzentrifugiert und die restliche Milch wurde mit einer Ausschlußmolekularmasse von 1 000 000 Da ultrafiltriert, wobei ein Modul der Firma Millipore verwendet wurde. Das dabei entstandene Permeat (8,5 L) wurde mit 100 ml NaOH auf pH 6,3 gebracht und die Trübung wurde wiederum abzentrifugiert. Das Permeat wurde im Anschluß nach herkömmlicher Methode bei 0,22 µm sterilfiltriert. Es mußten keine Vorfilter verwendet werden. Das Produkt war leicht sterilfiltrierbar. Im Anschluß wurde eine Analyse erstellt. Die Ergebnisse sind in Tabelle 2 gezeigt.

### Vergleichsbeispiel 4

20 L entfettete Kolostralmilch wurden ohne Vorbehandlung direkt bei einer Ausschlußmolekularmasse von 1 000 000 Da ultrafiltriert, wobei ein Modul der Firma Millipore verwendet wurde. Es wurden 10 L Permeat gewonnen. Diese wurden im Anschluß nach herkömmlicher Methode bei 0,22 µm sterilfiltriert. Es mußten keine Vorfilter verwendet werden. Das Produkt war leicht sterilfiltrierbar. Im Anschluß wurde eine Analyse erstellt. Die Ergebnisse sind in Tablle 2 gezeigt.

Bei dem Beispiel 2 und den Vergleichsbeispielen wurden nur die IGF-1- und die TGF-β-Konzentrationen und das Immunglobulin G bestimmt, die folgende Werte enthielten:

**Tabelle 2**

| | IGF-1 (ng/ml) | TGF-β (ng/ml) | IgG (mg/100 ml) |
|---|---|---|---|
| Beispiel 2 | 371 | 30 | 87 |
| Vergleichsbeispiel 1 | 81 | Spuren | 43 |
| Vergleichsbeispiel 2 | 48 | nicht nachweisbar | 25 |
| Vergleichsbeispiel 3 | 63 | nicht nachweisbar | 35 |
| Vergleichsbeispiel 4 | 23 | nicht nachweisbar | 15 |

Aus diesen Ergebnissen ergab sich eindeutig, daß eine pH-Absenkung mit anschließender Ultrafiltration, ohne daß der pH-Wert vor der Ultrafiltration wieder angehoben wird bessere Ergebnisse zeigt, als z.B. eine Ultrafiltration ohne pH-Absenkung. Es ergab sich ferner, daß eine pH-Absenkung auf ca. 4,5 ebenfalls kein zufriedenstellendes Ergebnis bringt, da sowohl bei pH 4,5 nicht alle Proteine in Lösung gehen, als auch bei der Entfernung des geronnenen Kaseins viele wertvolle Proteine mitgerissen werden. Weiterhin spielt die Temperatur ebenfalls eine wichtige Rolle, da eine Erhöhung der Temperatur eine Ultrafiltration nicht möglich macht, da das Kolostrum dabei verdickt.

Lediglich die in Beispiel 1 und 2 angegebenen Verfahren zeigten zufriedenstellende Ergebnisse.

### Vergleichsbeispiel 5

Zum weiteren Vergleich mit einem Produkt gemäß der vorliegenden Erfindung wurde von denselben Ausgangsmengen an Rohkolostrum wie in Beispiel 1 ausgegangen und wie oben beschrieben entfettet, das entfettete Rohkolostrum wurde dann jedoch auf einen pH-Wert von ca. 4,5 eingestellt und über einen Mikrofilter mit einer Porengröße von ca. 5 µm abgetrennt. Das erhaltene Molkefiltrat wurde dann mittels Zugabe von Natriumhydroxid auf einen pH-Wert von ca. 7 eingestellt und anschließend steril filtriert.

Die Analyse eines derartigen Kolostralmilchproduktes gemäß dem Stand der Technik ist in Tabelle 3 gezeigt.

Ein Vergleich der Wachstumsfaktorenfraktion zeigt einen etwa um 100% niedrigeren Gehalt an IGF-1 und nicht meßbare Werte an IGF-2 und TGF-β. Das erfindungsgemäße Kolostralmilchprodukt hat somit einen signifikant höheren Gehalt an den für die günstigen pharmakologischen und vitalen Wirkungen erforderlichen Wachstumsfaktoren und weist ferner einen deutlich höheren Immunglobulingehalt auf.

### Vergleichsbeispiel 5

**Tabelle 3**

| | |
|---|---|
| Energiegehalt | 80,0 kJ/100g |
| pH | 6,55 |
| Osmolarität | 418,0 mOsm/kg |
| Gesamtprotein | 0,43 g/100 ml |
| Trockensubstanz | 5,24 g/100 ml |
| Asche | 0,82 g/100 ml |
| Laktose | <0,5 g/100 ml |
| Glukose | 1,91 g/100 ml |
| Gesamtkohlenhydrat | 4,0 g/100 l |
| Fett | <0,02 g/100 ml |
| IgG | 0,039 g/100 ml |
| | |

| ***VITAMINE*** | |
|---|---|
| | |
| Thiamin (Vit. B1) | 40,0 µg/100 ml |
| Riboflavin (Vit. B2) | 180,0 µg/100 ml |
| Pyridoxin (Vit. B6) | 12,0 µg/100 ml |
| Cobalamin (Vit. B12) | 0,041 µg/100 ml |
| Folsäure | 4,2 µg/100 ml |
| Nicotinamid | 83,0 µg/100 ml |
| Panthotensäure | 300,0 µg/100 ml |
| | |

| ***IONEN*** | |
|---|---|
| | |
| Natrium (Na) | 1498,9 mg/l |
| Kalium (K) | 1599,2 mg/l |
| Kalzium (Ca) | 280,15 mg/l |
| Magnesium (Mg) | 109,88 mg/l |
| Eisen (Fe) | <0,22 mg/l |
| Kupfer (Cu) | <0,05 mg/l |
| Mangan (Mn) | <0,05 mg/l |
| Zink (Zn) | <0,05 mg/l |
| Chrom (Cr) | <0,01 mg/l |
| Phosphor (P) | 480,0 mg/l |
| Selen (Se) | 0,002 mg/l |
| | |

| ***ORGANISCHE MOLEKÜLE*** | |
|---|---|
| | |
| Kreatinin | 34,4 mg/l |
| Kreatin | 127,0 mg/l |
| | |

| ***FREIE AMINOSÄUREN*** | |
|---|---|
| | |
| Arginin | 5,6 mg/l |
| Tryptophan | <6,1 mg/l |
| Cystin | <0,7 mg/l |
| Methionin | 1,0 mg/l |
| Phosphoserin | 9,06 mg/l |
| Taurin | 115,0 mg/l |
| Phosphoethanolamin | 27,37 mg/l |
| Asparaginsäure | 2,66 mg/l |
| Threonin | 3,1 mg/l |
| Serin | 5,04 mg/l |
| Glutaminsäure | 37,0 mg/l |
| Glutamin | 7,31 mg/l |
| Prolin | 7,37 mg/l |
| Glycin | 4,85 mg/l |
| Alanin | 6,6 mg/l |
| Citrullin | 1,05 mg/l |
| Valin | 11,13 mg/l |
| Isoleucin | 4,85 mg/l |
| Leucin | 9,44 mg/l |
| Tyrosin | 3,26 mg/l |
| Phenylalanin | 4,95 mg/l |
| β-Alanin | <0,27 mg/l |
| β-Aminoisobuttersäure | 1,03 mg/l |
| Ornithin | 0,53 mg/l |
| Lysin | 7,99 mg/l |
| Histidin | 1,4 mg/l |
| | |

| ***NATÜRLICHE WACHSTUMS- FAKTOREN*** | |
|---|---|
| | |
| IGF-1 (Somatomedin C) | 70,0 ng/ml |
| IGF-2 | nicht erfaßbar |
| TGF-β | nicht erfaßbar |

### Beispiel 3

Das Kolostralmilchprodukt gemäß Beispiel 1 wird nach bekannten Verfahren in Liposomen eingeschlossen und in eine Cremegrundlage (im Beispielsfalle Soja-Öl, Tegomuls, Vitamin E + A, Cholesterol, HSA, Contrex, Carotin DF + HF, Orangenblüten-ÖI, Melissen-Öl, Mandarinen-Öl, Neroli-Öl und Aqua dest. mit einem Liposomengehalt von 0,1 ml/g (10%)) eingebracht.

Eine derartige Liposomencreme wurde verwendet, um ihre Einwirkungen auf die Wundheilung und auf neurodermitische Ekzeme zu untesuchen.

Wie bereits eingangs beschrieben, zeigte sich eine signifikante Besserung des gesamten dermatologischen Erscheinungsbildes der Neurodermitis in einer placebokontrollierten Cross-over-Studie, wobei in der Versuchsgruppe 20 Probanden teilnahmen und in der Kontrollgruppe 15 Probanden teilnahmen.

Somit steht mit der vorliegenden Erfindung erstmals ein Verfahren zu Verfügung, mit dem es möglich ist, die wichtigsten Bestandteile der Kolostralmilch nahezu quantitativ in ein Kolostralmilchprodukt zu überführen, welches dann hohe Gehalte an Wachstumsfaktoren und Immunglobulinen aufweist.

Selbstverständlich ist es möglich, gezielte Fraktionen, beispielsweise immunglobulinangereicherte Fraktionen und/oder Wachstumsfaktorfraktionen zu erhalten, die dann gegebenenfalls separat entweder oral appliziert werden können oder jedoch in Liposomen eingeschlossen werden können und dann in Form von Cremes, Pasten oder Salben oder Emulsionen auf die Haut aufgetragen werden können.

Erreicht wird eine derartige Fraktionierung des erfindungsgemäßen Kolostralmilchproduktes durch weitere Ultrafiltrationen mit definierten Ausschlußmolekularmassen von 300 kDa und/oder 150 kDa und/oder 100 kDa und/oder 50 kDa und/oder 30 kDa und/oder 20 kDa und/oder 10 kDa und/oder 5 kDa und/oder 1 kDa und/oder 0,5 kDa.

Weiterhin kann es vorteilhaft sein, den pH vor einer weiteren Fraktionierung wieder anzuheben, wenn man z.B. eine Entsalzung bei 1 kDA oder 0,5 kDa vornimmt, um die Moleküle wieder so groß zu machen, daß sie nicht mit durch den Filter gehen. Ob eine pH-Anhebung durchgeführt werden sollte oder nicht, hängt also weitgehend davon ab, was man mit weiteren Fraktionierungen erreichen will.

Welche Ausschlußmolekularmasse der Fachmann wählt, hängt ebenfalls davon ab, welche weiteren Fraktionen er aus dem erfindungsgemäßen Kolostralmilchprodukt erhalten möchte.

## Patentansprüche

1. Verfahren zur Herstellung eines wenigstens weitgehend entkaseinierten Kolostralmilchproduktes, wobei
Rohkolostrum entfettet wird;
derart angesäuert wird, daß Kasein in Lösung bleibt; und
eine Ultrafiltration mit einer Ausschlußmolekularmasse von ca. 10⁶ Da durchgeführt wird.

2. Verfahren nach Anspruch 1, **dadurch gekennzeichnet, daß** man das erhaltene Produkt mit weiteren Ultrafiltrationsschritten in unterschiedliche Molekularmassenbereiche fraktioniert.

3. Verfahren nach Anspruch 2, **dadurch gekennzeichnet, daß** man die weiteren Ultrafiltrationsschritte mit einer Ausschluß-molekularmasse von 300 kDa und/oder 150 kDa und/oder 100 kDa und/oder 50 kDa und/oder 30 kDa und/oder 20 kDa und/oder 10 kDa und/oder 5 kDa und/oder 1 kDa und/oder 0,5 kDa, durchführt.

4. Verfahren nach einem der Ansprüche 1 bis 3, **dadurch gekennzeichnet, daß** man den pH-Wert nach dem ersten Ultrafiltrationsschritt und nach den weiteren Ultrafiltrationsschritten auf den pH-Wert des Rohkolostrums, insbesondere auf einen pH von ca. 3,0 bis 6,9, vorzugsweise ca. 4,0 bis 5,5, besonders bevorzugt ca. 4,6 einstellt.

5. Verfahren nach einem der vorhergehenden Ansprüche, **dadurch gekennzeichnet, daß** man zum Ansäuern und/oder zur pH-Einstellung Salzsäure, Phosphorsäure, Milchsäure und/oder Zitronensäure verwendet.

6. Verfahren nach einem der vorhergehenden Ansprüche, **dadurch gekennzeichnet, daß** man die jeweils erhaltenen Produkte, insbesondere durch reverse Osmose, durch Ultrafiltration mit ca. 1 kDa oder ca. 0,5 kDa und/oder durch Dialyse entsalzt.

7. Verfahren nach einem der vorhergehenden Ansprüche, **dadurch gekennzeichnet, daß** man die Produkte entzuckert, wobei enzymatisch und/oder über Ultrafiltration entzuckert wird.

8. Verfahren nach einem der vorhergehenden Ansprüche, **dadurch gekennzeichnet, daß** man dem erhaltenen Produkt Additive, insbesondere Vitamine, Aromastoffe, Geschmacksverbesserer, Konservierungsstoffe usw. zusetzt.

9. Verfahren nach einem der vorhergehenden Ansprüche, **dadurch gekennzeichnet, daß** man die erhaltenen Produkte sterilisiert, insbesondere sterilfiltriert.

10. Wenigstens weitgehend entkaseiniertes Kolostralmilchprodukt,
**dadurch gekennzeichnet, daß**
es nach einem Verfahren gemäß wenigstens einem der Ansprüche 1 bis 9 erhältlich ist.

11. Kolostralmilchprodukt nach Anspruch 10, **dadurch gekennzeichnet, daß** es wenigstens ca. 75 ng/ml IGF-1 enthält.

12. Kolostralmilchprodukt nach Anspruch 10 oder 11, **dadurch gekennzeichnet, daß** es, bezogen auf das Rohkolostrum, ca. 50 bis 95%, insbesondere ca. 60 bis 95%, vorzugsweise ca. 70 bis 95%, besonders bevorzugt ca. 80 bis 95%, insbesondere bevorzugt wenigstens ca. 90% der im Rohkolostrum vorhandenen Wachstumsfaktoren enthält.

13. Kolostralmilchprodukt nach Anspruch 10 bis 12, **dadurch gekennzeichnet, daß** es bovinen oder equinen Ursprungs ist.

14. Kolostralmilchprodukt nach einem der Ansprüche 10 bis 13, **dadurch gekennzeichnet, daß** es in flüssiger, fester, pastöser oder in mikroverkapselter Form, insbesondere in Liposomen eingeschlossen, vorliegt.

15. Getränk, **dadurch gekennzeichnet, daß** es ein Kolostralmilchprodukt gemäß wenigstens einem der Ansprüche 10 bis 14 enthält.

16. Verwendung eines Kolostralmilchproduktes gemäß wenigstens einem der Ansprüche 10 bis 14 zur Herstellung eines Arzneimittels, Nahrungsergänzungsmittels oder Kosmetikums.

17. Verwendung eines Kolostralmilchproduktes gemäß wenigstens einem der Ansprüche 10 bis 14 als Zusatz für Arzneimittel, Nahrungsergänzungsmittel, Getränke oder Kosmetika.

18. Verwendung nach Anspruch 17, **dadurch gekennzeichnet, daß** das Kolostralmilchprodukt als Bestandteil von Babynahrung, diätetischer Nahrung, klinischer Nahrung, insbesondere Sondennahrung, verwendet wird.

19. Verwendung nach Anspruch 17, **dadurch gekennzeichnet, daß** das Kolostralmilchprodukt zur Herstellung eines Arzneimittels zur wenigstens unterstützenden Behandlung von Neurodermitis, Wundheilung, Erkrankungen des rheumatischen Formenkreises, Allergien, insbesondere Heuschnupfen, Pollenallergien; Herzinfarkten und muskulären Erkrankungen während der Rehabilitation, sowie zur unterstützenden Behandlung von Leber- und Knochenerkrankungen, verwendet wird.

20. Verwendung nach Anspruch 17, **dadurch gekennzeichnet, daß** das Kolostralmilchprodukt zur Herstellung eines Mittels zum Muskelzellschutz, zur Abkürzung der muskulären Erholungsphase, insbesondere im Sportbereich, zur Prophylaxe und Unterstützung viraler, bakterieller und mykotischer Infektionen, sowie zur Stärkung des Immunsystems, verwendet wird.

21. Verwendung nach Anspruch 17, **dadurch gekennzeichnet, daß** das Kolostralmilchprodukt als Zusatz zu Backwaren, insbesondere energiehaltigen Riegeln, Müsliriegeln oder Powerbars, verwendet wird.

## Claims

1. A process for preparing a colostral milk product which is at least largely decaseinated, wherein
raw colostrum is defatted;
acidified such that casein remains in solution; and
ultrafiltration with an exclusion molecular mass of approx. 10⁶ Da is performed.

2. The process according to claim 1, **characterized in that** the obtained product is fractionated into differenct molecular mass ranges through additional ultrafiltration steps.

3. The process according to claim 2, **characterized in that** the additional ultrafiltration steps are performed with an exclusion molecular mass of 300 kDa and/or 150 kDa and/or 100 kDa and/or 50 kDa and/or 30 kDa and/or 20 kDa and/or 10 kDa and/or 5 kDa and/or 1 kDa and/or 0.5 kDa.

4. The process according to any one of claims 1 to 3, **characterized in that** after the first ultrafiltration step and after the additional ultrafiltration steps, the pH value is adjusted to the pH value of the raw colostrum, in particular to a pH of approx. 3.0 to 6.9, preferably approx. 4.0 to 5.5, more preferably approx. 4.6.

5. The process according to any one of the preceding claims, **characterized by** use of hydrochloric acid, phosphoric acid, lactic acid and/or citric acid for acidification and for pH adjustment.

6. The process according to any one of the preceding claims, **characterized in that** the respective obtained products are desalted by reverse osmosis, by ultrafiltration with approx. 1 kDa or approx. 0.5 kDa and/or by dialysis.

7. The process according to any one of the preceding claims, **characterized in that** the products are desugarized, with desugarization being performed enzymatically and/or through ultrafiltration.

8. The process according to any one of the preceding claims, **characterized in that** additives, in particular vitamines, flavoring agents, flavor improving agents, preservatives, etc. are added to the obtained product.

9. The process according to any one of the preceding claims, **characterized in that** the obtained products are sterilized, in particular filtration sterilized.

10. A colostral milk product which is at least largely decaseinated,
**characterized in that**
it is obtainable through a process according to at least one of claims 1 to 9.

11. The colostral milk product according to claim 10, **characterized in that** it contains at least 75 ng/ml of IGF-1.

12. The colostral milk product according to claim 10 or 11, **characterized in that** relative to the raw colostrum it contains approx. 50 to 95%, in particular approx. 60 to 95%, preferably approx. 70 to 95%, more preferably approx. 80 to 95%, in a particularly preferred manner at least approx. 90% of the growth factors present in the raw colostrum.

13. The colostral milk product according to claim 10 to 12, **characterized in that** it is of bovine or equine origin.

14. The colostral milk product according to any one of claims 10 to 13, **characterized in that** it is present in liquid, solid, pasty or microencapsulated form, in particular enclosed in liposomes.

15. Beverage, **characterized in that** it contains a colostral milk product in accordance with at least one of claims 10 to 14.

16. Use of a colostral milk product according to at least one of claims 10 to 14 as a medicament, food supplement or cosmetic preparation.

17. Use of a colostral milk product according to at least one of claims 10 to 14 as an additive for medicaments, food supplements, beverages or cosmetic preparations.

18. Use according to claim 17, **characterized in that** the colostral milk product is used as an ingredient of baby food, dietetic food, clinical food, in particular food for tube feeding.

19. Use according to claim 17, **characterized in that** the colostral milk product is used as a medicament for at least supporting treatment of neurodermatitis, wound healing, rheumatoid conditions, allergies, in particular hay fever, pollen allergies; cardiac infarctions and muscle ailments during rehabilitation, and for supporting treatment of liver and bone ailments.

20. Use according to claim 17, **characterized in that** the colostral milk product is used for the protection of muscle cells, for shortening the muscular recovery period, for prophylaxis and support in viral, bacterial and mycotic infections, and for fortifying the immune system.

21. Use according to claim 17, **characterized in that** the colostral milk product is used as an additive for pastry and confectionery, in particular energy-containing bars, granola bars or power bars.

## Revendications

1. Procédé de production d'un produit tiré du lait colostral étant au moins en grande partie décaséiné, où
le colostrum cru est dégraissé;
acidifié de telle sorte que la caséine reste en solution; et où
une ultrafiltration avec une masse moléculaire d'exclusion d'environ 106 Da est exécutée.

2. Procédé selon la revendication 1, **caractérisé en ce que** l'on fractionne le produit obtenu avec d'autres étapes d'ultrafiltration dans différentes gammes de masses moléculaires.

3. Procédé selon la revendication 2, **caractérisé en ce que** l'on exécute les autres étapes d'ultrafiltration avec une masse moléculaire d'exclusion de 300 kDa et/ou 150 kDa et/ou 100 kDa et/ou 50 kDa et/ou 30 kDa et/ou 20 kDa et/ou 10 kDa et/ou 5 kDa et/ou 1 kDa et/ou 0,5 kDa.

4. Procédé selon l'une des revendications 1 à 3, **caractérisé en ce que** l'on ajuste la valeur pH après la première ultrafiltration et après les autres étapes d'ultrafiltration à la valeur pH du colostrum cru, en particulier à un pH d'env. 3,0 à 6,9, de manière préférentielle d'env. 4,0 à 5,5, de manière particulièrement préférée d'env. 4,6.

5. Procédé selon l'une des revendications précédentes, **caractérisé en ce que** l'on utilise de l'acide chlorhydrique, de l'acide phosphorique, de l'acide lactique et/ou de l'acide citrique pour acidifier et/ou pour ajuster le pH.

6. Procédé selon l'une des revendications précédentes, **caractérisé en ce que** l'on dessale chaque fois les produits obtenus en particulier par osmose inverse, par ultrafiltration avec env. 1 kDa ou env. 0,5 kDa et/ou par dialyse.

7. Procédé selon l'une des revendications précédentes, **caractérisé en ce que** l'on enlève le sucre des produits, ceci étant fait de manière enzymatique et/ou par ultrafiltration.

8. Procédé selon l'une des revendications précédentes, **caractérisé en ce que** l'on ajoute au produit obtenu des additifs, en particulier des vitamines, des arômes, des améliorateurs de goût, des agents conservateurs, etc.

9. Procédé selon l'une des revendications précédentes, **caractérisé en ce que** l'on stérilise les produits obtenus, en particulier par filtration stérilisatrice.

10. Produit tiré du lait colostral au moins en grande partie décaséiné
**caractérisé en ce que**
celui-ci est obtenu d'après un procédé selon au moins l'une des revendications 1 à 9.

11. Produit tiré du lait colostral selon la revendication 10, **caractérisé en ce qu'**il contient au moins env. 75 ng/ml IGF-1.

12. Produit tiré du lait colostral selon la revendication 10 ou 11, **caractérisé en ce que** celui-ci contient, par rapport au colostrum cru, env. 50 à 95%, en particulier env. 60 à 95%, de préférence env. 70 à 95%, de manière préférée env. 80 à 95%, de manière particulièrement préférée au moins env. 90% des facteurs de croissance présents dans le colostrum cru.

13. Produit tiré du lait colostral selon la revendication 10 à 12, **caractérisé en ce que** celui-ci est d'origine bovine ou chevaline.

14. Produit tiré du lait colostral selon l'une des revendications 10 à 13, **caractérisé en ce que** celui-ci est présent sous forme liquide, solide, pâteuse, microencapsulée, en particulier enfermé dans des liposomes.

15. Boisson, **caractérisée en ce que** celle-ci contient un produit tiré du lait colostral d'après au moins une des revendications 10 à 14.

16. Utilisation d'un produit tiré du lait colostral d'après au moins une des revendications 10 à 14 en tant que médicament, produit complémentaire d'alimentation ou produit cosmétique.

17. Utilisation d'un produit tiré du lait colostral d'après au moins une des revendications 10 à 14 en tant qu'additif pour des médicaments, des produits complémentaires d'alimentation, des boissons ou des produits cosmétiques.

18. Utilisation selon la revendication 17, **caractérisée en ce que** le produit tiré du lait colostral est utilisée en tant qu'ingrédient d'aliments pour bébés, d'aliments diététiques, d'aliments cliniques, en particulier pour l'alimentation par sonde.

19. Utilisation selon la revendication 17, **caractérisée en ce que** le produit tiré du lait colostral est utilisé en tant que médicament qui soutienne tout au moins le traitement des neurodermites, de la cicatrisation, des maladies rhumatismales, des allergies, en particulier du rhume des foins, des allergies au pollen; des infarctus du myocarde et des maladies musculaires pendant la réhabilitation, ainsi que pour soutenir le traitement des maladies du foie et des os.

20. Utilisation selon la revendication 17, **caractérisée en ce que** le produit tiré du lait colostral est utilisé pour la protection cellulaire des muscles, pour raccourcir la phase de repos musculaire, en particulier dans le domaine du sport, pour agir prophylactiquement et soutenir les traitements des infections virales, bactérielles et mycosiques, ainsi que pour fortifier le système immunitaire.

21. Utilisation selon la revendication 17, **caractérisée en ce que** le produit tiré du lait colostral est utilisé en tant qu'additif dans la pâtisserie, en particulier dans les barres énergétiques, les barres de muesli ou les powerbars.
